# NEUE EUROPÄISCHE PATENTSCHRIFT

(11) **EP 2 146 774 B2**
(45) Veröffentlichungstag und Bekanntmachung des Hinweises auf die Entscheidung über den Einspruch: **22.08.2018**
(45) Hinweis auf die Patenterteilung: 17.02.2016
(21) Anmeldenummer: 08748354.1
(22) Anmeldetag: 16.05.2008
(51) Int. Cl.: A61M 39/12, A61M 1/00

(54) **DRAINAGESCHLAUCHEINHEIT**
DRAINAGE TUBE UNIT
UNITÉ À TUYAU DE DRAINAGE

(30) Priorität: 22.05.2007 CH 823072007
(43) Veröffentlichungstag der Anmeldung: 27.01.2010
(73) Patentinhaber: Medela Holding AG, 6340 Baar (CH)
(72) Erfinder: RAMELLA, Ivo, CH-6030 Ebikon (CH); JODER, Fabian, CH-6340 Baar (CH)
(74) Vertreter: Clerc, Natalia
(86) Internationale Anmeldenummer: PCT/CH2008/000224
(87) Internationale Veröffentlichungsnummer: WO 2008/141470

(56) Entgegenhaltungen:
- EP-A1- 0 466 334
- EP-A1- 0 861 668
- WO-A-2007/128156
- WO-A1-01/24846
- WO-A1-01/34223
- WO-A1-2008/141471
- WO-A1-2008/141471
- WO-A2-97/48427
- WO-A2-03/057070
- WO-A2-2007/128156
- DE-A1- 19 913 713
- DE-A1- 19 913 713
- US-A- 5 328 456
- US-A- 5 582 588
- US-A1- 2005 061 025
- US-B1- 3 749 090
- US-B1- 3 889 675
- US-B1- 5 029 580
- US-B1- 5 134 996
- US-B1- 5 328 456
- US-B1- 5 582 588
- US-B1- 5 738 656
- US-B1- 6 626 827
- Deckblattb der Patentschrift EP 2146774
- USPTO Amtsbescheid
- Antwort auf USPTO Amtsbescheid

## Beschreibung

### Technisches Gebiet

Die Erfindung betrifft eine Drainageschlaucheinheit gemäss Oberbegriff des Patentanspruchs 1 bzw. 21 sowie ein Anschlussteil gemäss Oberbegriff des Patentanspruchs 17 und einen Endstecker gemäss Oberbegriff des Patentanspruchs 18.

### Stand der Technik

Zum Absaugen von Körperflüssigkeiten bzw. -fluiden im medizinischen Bereich, beispielsweise bei bzw. nach chirurgischen Eingriffen, aber auch in der Wunddrainage, der Thoraxdrainage oder beim Absaugen von Körperfetten, werden Drainagepumpsysteme eingesetzt. Diese Drainagepumpsysteme weisen üblicherweise eine Saugpumpe, einen oder mehrere Fluidsammelbehälter und eine Drainageschlauchverbindung zwischen Patient und Fluidsammelbehälter auf. Der Fluidsammelbehälter kann am Gehäuse der Drainagepumpe lösbar befestigt sein oder mit der Pumpe über einen Vakuumschlauch verbunden sein.

Indem mittels der Saug- bzw. Vakuumpumpe im Fluidsammelbehälter ein Unterdruck erzeugt wird, wird das Fluid oder Sekret von einer Kavität des Patienten über den Drainageschlauch in den Sammelbehälter gesaugt und dort gesammelt. Am pumpenseitigen Ausgang des Sammelbehälters angeordnete Filter schützen die Saugpumpe vor einer allfälligen Verunreinigung durch das abgesaugte Fluid. Ein derartiger Fluidsammelbehälter mit starrem Deckel und daran befestigtem flexiblen Beutel ist beispielsweise aus EP 0 861 668 und WO 01/24846 bekannt.

In EP 0 466 334 wird eine Drainageleitung mit einem Drainagekatheter und einer den Katheter umgebenden luftdichten Hülle offenbart. Der Katheter ist an seinen beiden Enden mit einem Anschlussteil versehen. Am patientenseitigen Anschlussteil ist ein Anschluss für einen Gasanalysator vorgesehen.

Es ist ferner bekannt, zusätzlich zur Drainageleitung eine Serviceleitung von der Pumpe zum Patienten zu führen. In US 5 738 656 wird beispielsweise ein doppellumiger Schlauch verwendet, wobei ein Lumen die Drainageleitung bildet und das zweite Lumen eine Luftleitung ist, welche am patientenseitigen Ende in die Drainageleitung mündet. Dadurch kann in die abzusaugende Kavität des Patienten Luft oder Gas zugeführt werden und so die Kavität gespült werden. Zudem kann dieses Lumen als Messleitung benützt werden, um Strömungs- oder Druckdifferenzen festzustellen. Dadurch lässt sich der Drainagevorgang optimal überwachen und auch automatisch steuern.

Aus WO 05/061025 wird eine mit dem patientenseitigen Ende des Drainageschlauchs verbundene Serviceleitung zum Spülen der Drainageleitung verwendet, um Verschlüsse der Leitung durch angesaugte Fluidklumpen oder Gewebe zu vermeiden bzw. zu beheben.

US 6 626 827 beschreibt eine Drainageschlaucheinheit mit zwei Schläuchen, welche am pumpenseitigen Ende ein y-förmiges Anschlussteil aufweist. Am patientenseitigen Ende münden die zwei Schläuche in zwei voneinander unabhängige Anschlussteile.

US 5 029 580 offenbart eine Drainageschlaucheinheit mit einem doppellumigen Schlauch, welcher eine Drainageleitung und eine Luftzufuhrleitung beinhaltet. Am patientenseitigen Ende weist der Schlauch innere Durchgangsöffnungen auf, welche die zwei Leitungen miteinander verbinden. Dieser Schlauch ist an seinen Enden mit einem pumpenseitigen bzw. einem patientenseitigen Anschlussteil versehen. In diesen Anschlussteilen sind ferner weitere Anschlussmöglichkeiten vorgesehen.

Auch US 5 134 996 offenbart einen mehrlumigen Drainageschlauch, welcher von einer Hülle umgeben ist und welcher an seinen zwei Enden mit Anschlussteilen versehen ist.

Diese Anschlüsse helfen dank ihrer Anschlussteile zwar, Fehlmanipulationen zu vermeiden. Sie sind jedoch relativ kompliziert aufgebaut, insbesondere da sie aus mehreren Einzelteilen bestehen. Zudem lassen sie sich nur mit einem doppellumigen Katheterschlauch, insbesondere nur mit einem Schlauch mit einem speziell ausgebildeten patientenseitigen Ende verwenden. Da diese Drainageschlaucheinheiten nicht mehrfach benützt werden können und als Einwegteile nach einmaligem Gebrauch weggeworfen werden, müssen sie jedoch so kostengünstig wie möglich sein.

WO 2007/128156, welche nachveröffentlicht ist, offenbart einen in einem Pumpengehäuse einsteckbaren Adapter für einen doppellumigen Schlauch.

### Darstellung der Erfindung

Es ist eine Aufgabe der Erfindung, eine Drainageschlaucheinheit zu schaffen, welche eine sichere Handhabung erlaubt und trotzdem kostengünstig herstellbar ist.

Diese Aufgabe löst eine Drainageschlaucheinheit mit den Merkmalen des Patentanspruchs 1.

Die erfindungsgemässe Drainageschlaucheinheit zum Absaugen von Körperfluiden mittels einer Saugpumpe weist einen Drainageschlauch zum Absaugen der Körperfluide und mindestens einen Serviceschlauch mit je einem patientenseitigen und einem pumpenseitigen Ende auf, wobei die patientenseitigen Enden des Drainageschlauchs und des Serviceschlauchs in einem gemeinsamen patientenseitigen Anschlussteil angeordnet sind und die pumpenseitigen Enden des Drainageschlauchs und des Serviceschlauchs in einem gemeinsamen pumpenseitigen, als Endstecker ausgebildeten, Anschlussteil angeordnet sind. Das pumpenseitige Anschlussteil ist zum Einstecken in eine zugehörige Absaugvorrichtung ausgebildet. Die Enden des Drainageschlauchs verlaufen getrennt von den Enden des Serviceschlauchs, wobei die patientenseitigen Enden in einen Drainagekanal und einen Servicekanal des patientenseitigen Anschlussteils münden und die pumpenseitigen Enden in einen Drainagekanal und einen Servicekanal des pumpenseitigen Anschlussteils münden.

Erfindungsgemäss lassen sich handelsübliche und kostengünstige einlumige Schläuche verwenden. Die Schläuche können, müssen aber nicht nebeneinander in die Anschlussteile gesteckt werden. Vorteilhaft an dieser Lösung ist, dass die Schlauchenden auch an verschiedenen Seiten der Anschlussteile in diese eingeführt sein können, so dass sich die Anschlussteile beliebig gestalten bzw. an die Gegebenheiten der Pumpe und der patientenseitigen Situation anpassen lassen. Die Schläuche können zudem zwischen den Anschlussteilen gesamthaft, über weite Strecken oder gar nicht parallel zueinander verlaufen.

Da eine bereits zusammengesteckte Schlaucheinheit mit zwei Endsteckem bzw. Anschlussteilen angeboten ist und da auf beiden Seiten Anschlussteile vorhanden sind, ist gewährleistet, dass die Schlaucheinheit korrekt in die zugehörige Absaugvorrichtung eingesteckt wird.

Vorzugsweise sind diese Anschlussteile kostengünstig aus Kunststoff im Spritzgussverfahren hergestellt und insbesondere einstückig gestaltet.

Das patientenseitige Anschlussteil lässt sich insbesondere dann im Spritzgussverfahren einstückig herstellen, wenn der Verbindungskanal auf einer Seite nach aussen offen ausgebildet ist und erst nachträglich verschlossen wird.

Erfolgt das Verschliessen mittels eines Stöpsels, so ist es vorteilhaft, auch diesen gleich einstückig im restlichen Anschlussteil anzuspritzen. Er lässt sich dadurch insbesondere gleich vor dem Auswerfen des Anschlussteils aus der Spritzgussform automatisch in die Öffnung des Verbindungskanals eindrücken.

Es ist eine weitere Aufgabe der Erfindung, eine Drainageschlaucheinheit zu schaffen, welche kostengünstig herstellbar ist.

Diese Aufgabe löst eine Drainageschlaucheinheit mit den Merkmalen des Patentanspruchs 21.

Die erfindungsgemässe Drainageschlaucheinheit zum Absaugen von Körperfluiden mittels einer Saugpumpe weist einen Drainageschlauch zum Absaugen der Körperfluide und mindestens einen Serviceschlauch mit je einem patientenseitigen und einem pumpenseitigen Ende auf, wobei die patientenseitigen Enden des Drainageschlauchs und des Serviceschlauchs in einem gemeinsamen patientenseitigen Anschlussteil angeordnet sind. Das patientenseitige Ende des Drainageschlauchs verläuft getrennt vom patientenseitigen Ende des Serviceschlauchs, wobei diese patientenseitigen Enden in einen Drainage- bzw. einen Servicekanal des patientenseitigen Anschlussteils münden, wobei der Servicekanal einen kleineren Durchmesser aufweist als der Drainagekanal und wobei die zwei Kanäle über einen Verbindungskanal miteinander verbunden sind.

Da die Verbindung zwischen den zwei Leitungen im patientenseitigen Anschlussteil und nicht mehr im Schlauch selber erfolgt, lassen sich einfache, handelsübliche einlumige Schläuche, insbesondere aus Silikon oder PVC, verwenden. Dieses Anschlussteil lässt sich auch ohne pumpenseitiges Anschlussteil in einem Schlauchsystem einsetzen.

In allen Ausführungsformen können anstelle von zwei Leitungen auch mehrere Leitungen verwendet werden, so dass in den Anschlussteilen jeweils nicht nur ein Servicekanal sondern mehrere Servicekanäle vorhanden sind, wobei mindestens einer dieser Kanäle patientenseitig mit dem Drainagekanal über den Verbindungskanal verbunden ist.

Weitere vorteilhafte Ausführungsformen gehen aus den abhängigen Patentansprüchen hervor.

### Kurze Beschreibung der Zeichnungen

Im Folgenden wird der Erfindungsgegenstand anhand eines bevorzugten Ausführungsbeispiels, welches in den beiliegenden Zeichnungen dargestellt ist, erläutert. Es zeigen:
- Figur 1: eine perspektivische Darstellung einer erfindungsgemässen Drainageschlaucheinheit;
- Figur 2: eine perspektivische Darstellung durch eine in Längsrichtung geschnittene Drainageschlaucheinheit gemäss Figur 1;
- Figur 3: einen Längsschnitt durch die Drainageschlaucheinheit gemäss Figur 1 und
- Figur 4: eine perspektivische Darstellung einer Drainagepumpvorrichtung mit einem pumpenseitigen Anschlussteil der erfindungsgemässen Drainageschlaucheinheit.

### Wege zur Ausführung der Erfindung

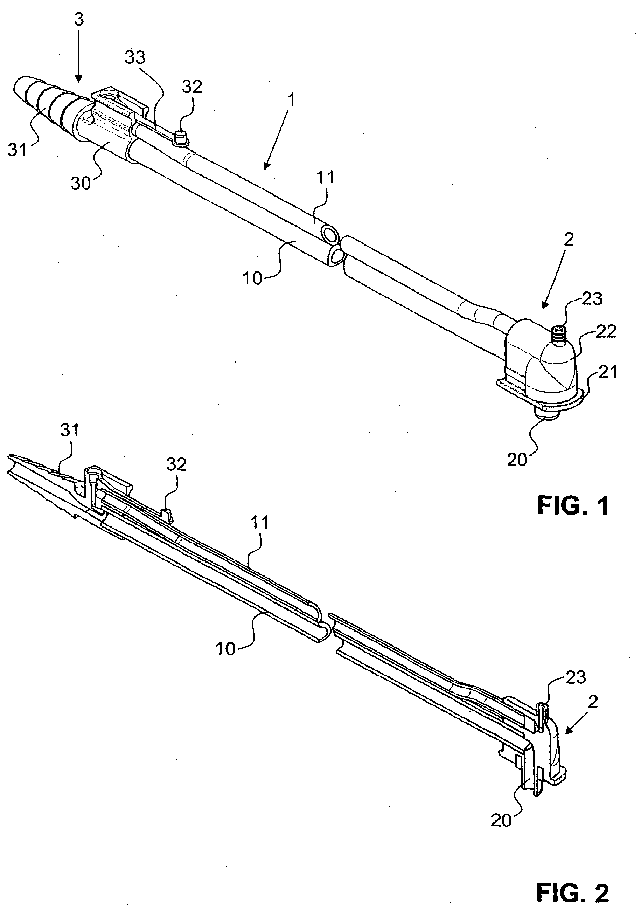

In Figur 1 ist die erfindungsgemässe Drainageschlaucheinheit dargestellt, wie sie in den eingangs erwähnten Drainageabsaugvorrichtungen eingesetzt wird. Sie besteht im Wesentlichen aus einem Schlauchsystem 1 mit zwei oder mehreren Schläuchen 10, 11, einem pumpenseitigen Anschlussteil 2 und einem patientenseitigen Anschlussteil 3.

Die Schläuche 10, 11 sind vorzugsweise einlumige, voneinander unabhängige Schläuche. Vorzugsweise sind sie aus Silikon oder PVC gefertigt. Sie verlaufen an ihren Enden getrennt voneinander. Dazwischen können sie aneinander geklebt, miteinander verschweisst oder anderweitig miteinander verbunden sein. In den Figuren sind die Schläuche nicht in ihrer gesamten Länge, sondern unterbrochen dargestellt.

Vorzugsweise weisen die zwei Schläuche unterschiedliche Durchmesser auf. Der dickere Schlauch 10 bildet dabei eine Unterdruck- und Drainageleitung zum Absaugen des Körperfluids. Der dünnere Schlauch 11 bildet eine Serviceleitung, welche beispielsweise die oben beschriebene oder eine ähnliche Druckmessung und/oder eine Reinigung der Drainageleitung ermöglicht. Beide Anwendungen sind gemeinsam aber zeitlich nacheinander möglich, wenn die Absaugeinheit am pumpenseitigen Ende der Serviceleitung beispielsweise über ein Ventil verfügt, welches für die Unterdruckmessung während des Absaugvorgangs geschlossen ist. Während des Reinigungsmodus ist das Ventil jedoch geöffnet. Die Serviceleitung kann auch auf andere bekannte Arten eingesetzt werden. Die Servicefunktion dient zur Unterstützung der Drainagefunktion und kann beispielsweise die eingangs erwähnten Funktionen gemäss dem Stand der Technik umfassen.

Die zwei Schläuche 10, 11 verlaufen vorzugsweise über annähernd die gesamte Länge parallel zueinander, insbesondere ihre Enden münden parallel, aber beabstandet zueinander in die jeweiligen Anschlussteile bzw. -elemente 2, 3. Unter beabstandet wird verstanden, dass sie aneinander anliegen können oder dass sie einen Freiraum zwischen sich lassen können. Sie ragen mindestens in einem der zwei Teile auf derselben Seite des Anschlussteils hinein. Die Enden sind in den Anschlussteilen 2, 3 eingesteckt, in ihnen verklebt oder anderweitig befestigt.

Im Folgenden wird zuerst das pumpenseitige Anschlussteil 2 beschrieben, welches als Endstecker zum Einstecken in die zugehörige Absaugvorrichtung ausgebildet ist. Pumpenseitig meint in diesem Zusammenhang jedoch lediglich patientenfern. Das Anschlussteil kann anstatt in einem Pumpengehäuse auch in einem Fluidsammelbehälter oder einer anderen patientenfernen Einheit angeordnet sein. Wird deshalb im Folgenden und in den Patentansprüchen von pumpenseitig gesprochen, ist ebenso behälterseitig gemeint. Wird im Folgenden und in den Patentansprüchen von Absaugvorrichtung gesprochen, ist unter anderem das Gehäuse der Pumpe oder der Fluidsammelbehälter gemeint.

Das pumpenseitige Anschlussteil 2 ist vorzugsweise aus Kunststoff im Spritzgussverfahren gefertigt und es ist vorzugsweise einstückig ausgebildet.

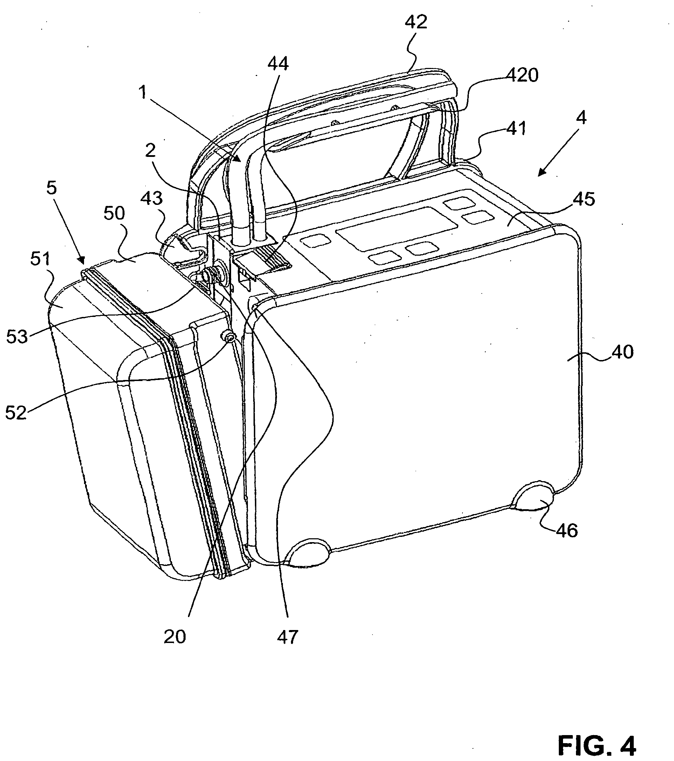

Es weist einen im Wesentlichen quaderförmigen Grundkörper 2 auf, welcher hier mit einem umlaufenden Flansch 21 versehen ist. Mit diesem Flansch 21 lässt sich das Teil 2 formschlüssig in eine entsprechende Aufnahme des Pumpengehäuses einführen und halten, wie in Figur 4 gezeigt ist.

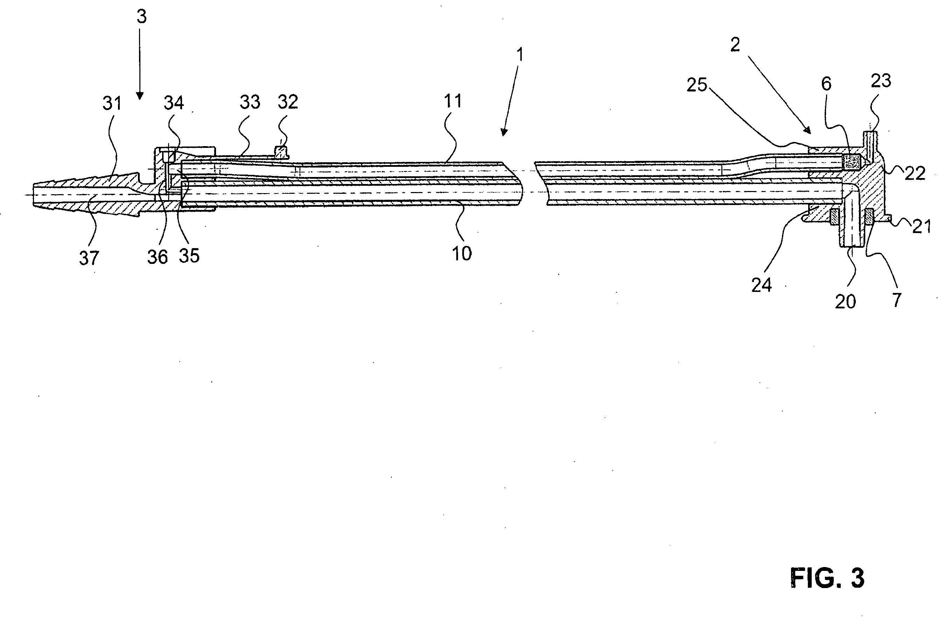

Wie in den Figuren 2 und 3 erkennbar ist, weist das Anschlussteil 2 zwei Kanäle 24, 25 auf, in deren parallel zueinander, aber voneinander beabstandet verlaufenden Mündungsöffnungen die pumpenseitigen Enden des Drainage- und Serviceschlauchs 10, 11 eingesteckt sind.

Im pumpenseitigen Servicekanal 25 ist ein Filter 6 angeordnet. Es handelt sich beispielsweise um ein hydrophobes und/oder um eine Bakterienfilter. Anschliessend verjüngt sich der Servicekanal 25 und biegt sich rechtwinklig zur Mündung. Er endet in einem dem Grundkörper 22 in Form eines Anschlussstutzens vorstehenden Serviceeingang 23. Dieser Serviceeingang 23 dient zur Verbindung mit einer Serviceeinheit der Saugvorrichtung.

Der pumpenseitige Drainagekanal 24 biegt sich ebenfalls rechtwinklig zu seiner Mündung und endet ebenfalls in einem dem Grundkörper 22 vorstehenden Anschlussstutzen, dem pumpenseitigen Drainageausgang 20. Dieser Ausgang 20 dient zur Verbindung mit einem Fluidsammelbehälter. Der Ausgang 20 ist hier rechtwinklig zur Mündung des Schlauchs 10 angeordnet, er kann aber auch auf einer anderen, von der Mündungsseite unterschiedlichen Seite des Grundkörpers 22 angeordnet sein. Dasselbe gilt für den Serviceeingang 23 in Bezug auf die Mündung des Serviceschlauchs 10.

Durch den Drainageausgang 20 gelangt das abgesaugte Fluid in den Behälter. Damit Dichtheit gewährleistet ist, kann im Grundkörper 22 eine umlaufende Nut um den Drainageausgang 20 vorgesehen sein. Die Nut kann mit einem Dichtring versehen sein. Vorzugsweise ist der Drainageausgang 20 auf einer Seite des Grundkörpers 22 angeordnet, welche der Seite mit dem Serviceeingang 23 gegenüberliegt.

Das patientenseitige Anschlussteil 3 ist ebenfalls vorzugsweise aus Kunststoff im Spritzgussverfahren hergestellt. Auch es ist vorzugsweise einstückig ausgebildet.

Es weist einen Grundkörper 30 mit zwei parallel zueinander aber beabstandet voneinander verlaufenden Mündungen für die patientenseitigen Enden des Drainageschlauchs 10 und des Serviceschlauchs 11 auf. An diesem Grundkörper 30 ist ein patientenseitiger Drainageeingang 31 angeformt, welcher kegelförmig gestuft ausgebildet ist und sich zu seinem freien offenen Ende hin verjüngt. Er weist im Querschnitt eine Tannenbaumform auf. Der Drainageeingang 31 verläuft dabei vorzugsweise annähernd in axialer Flucht zur Mündung des patientenseitigen Endes des Drainageschlauchs 10, so dass der patientenseitige Drainagekanal 37 im Innern des Anschlussteils annähernd geradlinig verläuft.

Das patientenseitige Ende des Serviceschlauchs 11 steckt in einer Mündung eines patientenseitigen Servicekanals 35, welcher vorzugsweise einen kleineren Durchmesser aufweist als der Drainagekanal 37. Der Kanal 37 weist, wie übrigens alle anderen beschriebenen Kanäle, eine Stufe auf, welche als Anschlag für den Schlauch 11 dient. Die oben beschriebenen Mündungen werden als sich bis zu diesen Stufen erstreckend verstanden.

Der Servicekanal 23 endet im Grundkörper 30 und mündet dort in einen Verbindungskanal 36, welcher den Servicekanal 23 vorzugsweise senkrecht streift. Der Verbindungskanal 36 weist denselben oder vorzugsweise einen kleineren Durchmesser auf als der Servicekanal 35. Er endet einerseits in einem rechtwinkligen Bogen im Drainagekanal 37, vorzugsweise bei dessen Stufe zur Mündung hin. Sein anderes Ende bildet eine Öffnung 34 nach aussen, welche vorzugsweise senkrecht zu den Mündungen im Grundkörper 30 angeordnet ist.

Diese Öffnung 34 ist mit einem Verschlussdeckel 32, hier ein Stöpsel, verschlossen. In der Figur ist er noch im offenen Zustand dargestellt, wobei er vorzugsweise in dieser Konfiguration bereits geschlossen ist. Vorzugsweise wird er nämlich bereits beim Auswerfen aus der Spritzgussmaschine geschlossen, also lange bevor die Schläuche 10, 11 befestigt werden.

Vorzugsweise ist der Verschlussdeckel 32 einstückig mit dem restlichen Anschlussteil 3 hergestellt und ist deshalb, wie hier gezeigt, über ein Band 33 mit dem Grundkörper 30 verbunden. Diese Öffnung erlaubt die einstückige Herstellung dieses Anschlussteils.

In Figur 4 ist eine Drainagepumpvorrichtung dargestellt, mit welcher die erfindungsgemässe Drainageschlaucheinheit vorzugsweise verwendet wird. Sie dient zum Absaugen von Körperflüssigkeiten bzw. -fluiden im medizinischen Bereich, beispielsweise bei bzw. nach chirurgischen Eingriffen, aber auch zur Wunddrainage, zur Thoraxdrainage oder zum Absaugen von Körperfetten.

Die Schlaucheinheit kann jedoch auch mit anderen Drainagepumpvorrichtungen eingesetzt werden. Es ist zwar bevorzugt, aber nicht notwendig, dass dabei der Fluidsammelbehälter und die Pumpeinheit ohne weitere Zwischenleitungen mittels des pumpenseitigen Anschlussteils miteinander verbunden sind.

Die hier dargestellte Drainagepumpvorrichtung weist ein Pumpengehäuse 4 auf, in welcher eine Vakuum- oder Saugpumpe und Elektronik zum Betrieb der Pumpe bzw. zum Auswerten von dank des Serviceschlauchs erhaltenen Messwerten angeordnet ist.

Das Pumpengehäuse 4 ist vorzugsweise quaderförmig ausgebildet und weist eine vordere Wand 40, eine Rückwand 41, einen Tragbügel 42 und Füsse 46 auf. Auf einer oberen Seitenwand des Gehäuses 4 ist ein Bedienfeld 45 zur Bedienung der Pumpe, vorzugsweise mit einem Display, angeordnet.

An einer seitlichen Seitenwand stehen die vordere Wand 40 und die Rückwand 41 vor und bilden eine Aufnahme für einen Fluidsammelbehälter 5. Dieser Fluidsammelbehälter 5 besteht hier vorzugsweise aus zwei Behälterhälften 50, 51 und ist aus einem durchsichtigen Kunststoff hergestellt.

Der Behälter 5 lässt sich lösbar am Pumpengehäuse 4 befestigen, vorzugsweise wird er dabei eingeschwenkt und rastet in dieser Position ein. Hierzu weisen die vordere Wand 40 und die Rückwand 41 des Pumpengehäuses 4 obere und untere Kulissenführungen auf, in welche obere und untere Befestigungsbolzen 52 des Behälters 5 eingreifen. In der Figur ist nur ein oberer Bolzen sichtbar. Die unteren Bolzen sind, wie anhand der Schrägstellung des Behälters 5 erkennbar ist, bereits eingerastet.

Der Behälter 5 weist einen Haken 53 auf, welcher zum Gehäuse 4 hin gerichtet ist und in welchen eine Kipptaste 44 des Gehäuses 4 mit einem entsprechenden Vorsprung eingreift. Dadurch ist der Behälter 5 lösbar am Gehäuse 4 fixiert.

Am Gehäuse 4 ist, dem Behälter 5 zugewandt, ein Sauganschluss 47 vorhanden. Er weist die Form eines Stutzens auf, welcher in eine entsprechende Öffnung des Behälters 5 eingreift. Dadurch lässt sich im Behälter 5 mittels der Saugpumpe ein Unterdruck erzeugen.

Im Gehäuse 4 ist ferner eine im wesentlichen quaderförmige Ausnehmung vorhanden, in welche das pumpenseitige Anschlussteil 2 der erfindungsgemässen Drainageschlaucheinheit einsteckbar ist und in diesem formschlüssig lösbar gehalten ist. Der behälterseitige Drainageausgang 20 des Anschlussteils 2 ist dabei zum Behälter 5 hingerichtet. Durch ihn gelangt das abgesaugte Fluid in den Behälter 5.

Rechtwinklig dazu mündet das Schlauchsystem 1 mit den zwei Schläuchen 10, 11 in das pumpenseitige Anschlussteil 2. Das Schlauchsystem 1 ist in diesem Beispiel entlang des Gehäuses 4 geführt in einer am Tragbügel 42 angeordneten Rinne 420 gehalten.

Nicht erkennbar ist der Serviceeingang 23, welcher in das Pumpengehäuse 4 hineinragt und mit einer entsprechenden Steuerungs- und/oder Auswerteinheit verbunden ist.

Die erfindungsgemässe Drainageschlaucheinheit ist kostengünstig herstellbar und einfach und sicher im Gebrauch.

### Bezugszeichenliste

- 1: Schlauchsystem
- 10: Drainageschlauch
- 11: Serviceschlauch

- 2: pumpenseitiges Anschlussteil
- 20: pumpenseitiger Drainageausgang
- 21: Flansch
- 22: Grundkörper
- 23: pumpenseitiger Serviceeingang
- 24: pumpenseitiger Drainagekanal
- 25: pumpenseitiger Servicekanal

- 3: patientenseitiges Anschlussteil
- 30: Grundkörper
- 31: patientenseitiger Drainageeingang
- 32: Verschlussdeckel
- 33: Band
- 34: Öffnung
- 35: patientenseitiger Servicekanal
- 36: Verbindungskanal
- 37: patientenseitiger Drainagekanal

- 4: Pumpengehäuse
- 40: vordere Wand
- 41: Rückwand
- 42: Tragbügel

## Patentansprüche

1. Drainageschlaucheinheit zum Absaugen von Körperfluiden mittels einer Saugpumpe, wobei die Schlaucheinheit einen Drainageschlauch (10) zum Absaugen der Körperfluide und mindestens einen Serviceschlauch (11) mit je einem patientenseitigen und einem pumpenseitigen Ende aufweist, wobei die patientenseitigen Enden des Drainageschlauchs (10) und des Serviceschlauchs (11) in einem gemeinsamen patientenseitigen Anschlussteil (3) angeordnet sind und die pumpenseitigen Enden des Drainageschlauchs (10) und des Serviceschlauchs (11) in einem gemeinsamen pumpenseitigen Endstecker (2) angeordnet sind, wobei der pumpenseitige Endstecker (2) zum Einstecken in eine zugehörige Absaugvorrichtung ausgebildet ist, wobei die Enden des Drainageschlauchs (10) getrennt von den Enden des Serviceschlauchs (11) verlaufen, und wobei das patientenseitige Ende des Drainageschlauchs (10) in einen Drainagekanal (37) des patientenseitigen Anschlussteils (3) mündet und das patientenseitige Ende des Serviceschlauchs (11) in einen Servicekanal (35) des patientenseitigen Anschlussteils (3) mündet und das pumpenseitige Ende des Drainageschlauchs (10) in einen Drainagekanal (24) des pumpenseitigen Endsteckers (2) mündet und das pumpenseitige Ende des Serviceschlauchs (11) in einen Servicekanal (25) des pumpenseitigen Endsteckers (2) mündet.

2. Drainageschlaucheinheit nach Anspruch 1, wobei der pumpenseitige Endstecker (2) und/oder das patientenseitige Anschlussteil (3) einstückig ausgebildet sind.

3. Drainageschlaucheinheit nach einem der Ansprüche 1 oder 2, wobei die patienten- und pumpenseitigen Enden des Drainage bzw. des Serviceschlauchs (11) parallel zueinander verlaufend in den Endstecker (2) und dem patientenseitigen Anschlussteil (3) münden.

4. Drainageschlaucheinheit nach einem der Ansprüche 1 bis 3, wobei die zwei Schläuche zwei separate einlumige Schläuche sind.

5. Drainageschlaucheinheit nach einem der Ansprüche 1 bis 4, wobei die zwei Schläuche mindestens im Endstecker (2) und/oder im patientenseitigen Anschlussteil (2) auf derselben Seite des Endsteckers (2) bzw. des patientenseitigen Anschlussteils (2) in diesen bzw. dieses münden.

6. Drainageschlaucheinheit nach einem der Ansprüche 1 bis 5, wobei der Drainagekanal (24) des Endsteckers (2) eine Drainagemündung zur Aufnahme des pumpenseitigen Endes des Drainageschlauchs (10) und einen Drainageausgang (20) aufweist, wobei der Drainageausgang (20) an einer anderen Seite als die Drainagemündung angeordnet ist.

7. Drainageschlaucheinheit nach Anspruch 6, wobei der Endsteckers (2) im Wesentlichen quaderförmig ausgebildet ist und wobei der Drainageausgang (20) im Wesentlichen rechtwinklig zur Drainagemündung angeordnet ist.

8. Drainageschlaucheinheit nach einem der Ansprüche 1 bis 7, wobei der Servicekanal (25) des Endsteckers (2) eine Servicemündung zur Aufnahme des pumpenseitigen Endes des Serviceschlauchs (11) und einen Serviceeingang (23) aufweist, wobei der Serviceeingang (23) an einem anderen Ende als die Servicemündung angeordnet ist.

9. Drainageschlaucheinheit nach Anspruch 8, wobei der Endstecker (2) im Wesentlichen quaderförmig ausgebildet ist und wobei der Serviceeingang (23) im Wesentlichen rechtwinklig zur Servicemündung angeordnet ist.

10. Drainageschlaucheinheit nach Anspruch 8 und 9, wobei der Serviceeingang (23) an einer dem Drainageausgang (20) gegenüberliegenden Seite des Endsteckers (2) angeordnet ist.

11. Drainageschlaucheinheit nach einem der Ansprüche 1 bis 10, wobei im Servicekanal (25) des Endsteckers (2) ein Filter (6) angeordnet ist.

12. Drainageschlaucheinheit nach einem der Ansprüche 1 bis 11, wobei der Endsteckers (2) so ausgebildet ist, dass ers formschlüssig lösbar in der Saugpumpe haltbar ist.

13. Drainageschlaucheinheit nach einem der Ansprüche 1 bis 12, wobei der Drainagekanal (37) und der Servicekanal (35) des patientenseitigen Anschlussteils (3) über einen Verbindungskanal (36) miteinander verbunden sind.

14. Drainageschlaucheinheit nach Anspruch 13, wobei der Verbindungskanal (36) an einem ersten Ende in den Drainagekanal (37) und an einem zweiten Ende nach aussen führt, wobei dieses zweite Ende mit einem Verschlussdeckel (32) verschliessbar ist.

15. Drainageschlaucheinheit nach Anspruch 14, wobei der Verschlussdeckel (32) einstückig am patientenseitigen Anschlussteil (3) angeformt ist.

16. Drainageschlaucheinheit nach einem der Ansprüche 1 bis 15, wobei das patientenseitige Anschlussteil (3) einen Grundkörper (30) zur Aufnahme der Enden des Drainage- und des Serviceschlauchs (10, 11) und einen am Grundkörper (30) anschliessenden kegelförmig und gestuft ausgebildeten patientenseitigen Drainageeingang (31) aufweist, wobei der Drainageeingang (31) annähernd in axialer Flucht zur Mündung des patientenseitigen Endes des Drainageschlauchs (10) verläuft.

17. Patientenseitiges Anschlussteil (3) zur Verwendung in einer Drainageschlaucheinheit gemäss einem der Ansprüche 1 bis 16, wobei das patientenseitige Anschlussteil (3) einen Drainagekanal (37) mit einem patientenseitigen Drainageeingang (31) und mit einer pumpenseitigen Drainagemündung zur Aufnahme eines patientenseitigen Endes eines Drainageschlauchs (10) und einen pumpenseitigen Servicekanal (35) zur Aufnahme eines vom patientenseitigen Ende des Drainageschlauchs (10) getrennt verlaufenden Endes eines Serviceschlauchs (11) aufweist, wobei der Servicekanal (35) des patientenseitigen Anschlussteils (3) einen kleineren Durchmesser aufweist als der Drainagekanal (37) des patientenseitigen Anschlussteils (3) und wobei ferner ein Verbindungskanal (36) vorhanden ist, welcher den Servicekanal (35) des patientenseitigen Anschlussteils (3) mit dem Drainagekanal (37) des patientenseitigen Anschlussteils (3) verbindet, wobei der Verbindungskanal (36) an einem ersten Ende in den Drainagekanal (37) des patientenseitigen Anschlussteils (3) und an einem zweiten Ende nach aussen führt, wobei dieses zweite Ende verschliessbar ausgebildet ist und wobei das Anschlussteil einstückig im Spritzgussverfahren hergestellt ist.

18. Pumpenseitiger Endstecker zur Verwendung in einer Drainageschlaucheinheit gemäss einem der Ansprüche 1 bis 16, wobei der Endstecker (2) einen Grundkörper (22) aufweist, in welchem ein Drainagekanal (24) des Endsteckers (2) und ein Servicekanal (25) des Endsteckers (2) verlaufen, wobei der Drainagekanal (24) einen pumpenseitigen Drainageausgang (20) zur Verbindung mit einer zugehörigen Absaugvorrichtung und eine patientenseitige Drainagemündung zur Aufnahme eines pumpenseitigen Endes eines Drainageschlauchs (10) aufweist, wobei der Drainageausgang (20) an einer anderen Seite des Grundkörpers (22) angeordnet ist als die Drainagemündung, und wobei der Servicekanal (25) des Endsteckers (2) einen pumpenseitigen Serviceeingang (23) zur Verbindung mit einer zugehörigen Absaugvorrichtung und eine Servicemündung zur Aufnahme eines vom pumpenseitigen Ende des Drainageschlauchs (10) getrennt verlaufenden pumpenseitigen Endes des Serviceschlauchs (11) aufweist, wobei der Serviceeingang (23) an einer anderen Seite des Grundkörpers (22) angeordnet ist als die Servicemündung und wobei der Endstecker (2) zum Einstecken in die Absaugvorrichtung ausgebildet ist.

19. Pumpenseitiger Endstecker nach Anspruch 18, wobei der Grundkörper (22) im wesentlichen quaderförmig ausgebildet ist, wobei der Drainageausgang (20) im Wesentlichen rechtwinklig zur Drainagemündung angeordnet ist und wobei der Serviceeingang (23) im Wesentlichen rechtwinklig zur Servicemündung angeordnet ist.

20. Pumpenseitiger Endstecker nach Anspruch 19, wobei der Drainageausgang (20) an einer gegenüberliegenden Seite zum Serviceeingang (23) angeordnet ist.

21. Drainageschlaucheinheit zum Absaugen von Körperfluiden mittels einer Saugpumpe, wobei die Schlaucheinheit einen Drainageschlauch (10) zum Absaugen der Körperfluide und mindestens einen Serviceschlauch (11) mit je einem patientenseitigen und einem pumpenseitigen Ende aufweist, wobei die patientenseitigen Enden des Drainageschlauchs (10) und des Serviceschlauchs (11) in einem gemeinsamen patientenseitigen Anschlussteil (3) nach Anspruch 17 angeordnet sind, wobei das patientenseitige Ende des Drainageschlauchs (10) getrennt vom patientenseitigen Ende des Serviceschlauchs (11) verläuft, und wobei das patientenseitige Ende des Drainageschlauchs (10) in einen Drainagekanal (37) des patientenseitigen Anschlussteils (3) nach Anspruch 17 mündet und das patientenseitige Ende des Serviceschlauchs (11) in einen Servicekanal (35) des patientenseitigen Anschlussteils (3) nach Anspruch 17 mündet.

## Claims

1. Drainage tube unit for aspirating body fluids by means of a suction pump, the tube unit comprising a drainage tube (10) for aspirating the body fluids and at least one service tube (11), each of them with a patient-side end and a pump-side end, the patient-side ends of the drainage tube (10) and of the service tube (11) being arranged in a common patient-side attachment part (3), and the pump-side ends of the drainage tube (10) and of the service tube (11) being arranged in a common pump-side end-connector (2), the pump-side end-connector (2) being designed to be plugged into an associated suction device, wherein the ends of the drainage tube (10) extend separate from the ends of the service tube (11), and wherein the patient-side end of the drainage tube (10) opens into a drainage channel (37) of the patient-side attachment part (3) and the patient-side end of the service tube (11) opens into a service channel (35) of the patient-side attachment part (3) and the pump-side end of the drainage tube (10) opens into a drainage channel (24) of the pump-side end-connector (2) and the pump-side end of the service tube (11) opens into a service channel (25) of the pump-side end-connector (2).

2. Drainage tube unit according to Claim 1, in which the pump-side end-connector (2) and/or the patient-side attachment part (3) are designed in one piece.

3. Drainage tube unit according to one of Claims 1 and 2, in which the patient-side and pump-side ends of the drainage tube and service tube (11) extend parallel to each other as they open into the end-connector (2) and the patient-side attachment part (3).

4. Drainage tube unit according to one of Claims 1 to 3, in which the two tubes (10, 11) are two separate single-lumen tubes.

5. Drainage tube unit according to one of Claims 1 to 4, in which the two tubes (10, 11), at least in the end-connector (2) and/or in the patient-side attachment part (2), open into the end-connector (2) or into the patient-side attachment part (2), respectively, on the same side of the end-connector (2) or of the attachment part (2, 3), respectively.

6. Drainage tube unit according to one of Claims 1 to 5, in which the drainage channel (24) of the end-connector (2) has a drainage mouth, for receiving the pump-side end of the drainage tube (10), and a drainage outlet (20), which drainage outlet (20) is arranged on a different side than the drainage mouth.

7. Drainage tube unit according to Claim 6, in which the end-connector (2) has a substantially cuboid shape, and in which the drainage outlet (20) is arranged substantially at right angles to the drainage mouth.

8. Drainage tube unit according to one of Claims 1 to 7, in which the service channel (25) of the end-connector (2) has a service mouth, for receiving the pump-side end of the service tube (11), and a service inlet (23), which service inlet (23) is arranged on a different end than the service mouth.

9. Drainage tube unit according to Claim 8, in which the end-connector (2) has a substantially cuboid shape, and in which the service inlet (23) is arranged substantially at right angles to the service mouth.

10. Drainage tube unit according to Claims 8 and 9, in which the service inlet (23) is arranged on a side of the end-connector (2) lying opposite the drainage outlet (20).

11. Drainage tube unit according to one of Claims 1 to 10, in which a filter (6) is arranged in the service channel (25) of the end-connector (2).

12. Drainage tube unit according to one of Claims 1 to 11, in which the end-connector (2) is designed such that it is able to be held releasably in the suction pump with a form fit.

13. Drainage tube unit according to one of Claims 1 to 12, in which the drainage channel (37) and the service channel (35) of the patient-side attachment part (3) are connected to each other via a connection channel (36).

14. Drainage tube unit according to Claim 13, in which a first end of the connection channel (36) leads into the drainage channel (37) and a second end leads to the outside, which second end is able to be closed with a sealing cover (32).

15. Drainage tube unit according to Claim 14, in which the sealing cover (32) is formed integrally on the patient-side attachment part (3).

16. Drainage tube unit according to one of Claims 1 to 15, in which the patient-side attachment part (3) has a main body (30) for receiving the ends of the drainage tube and service tube (10, 11), and, adjoining the main body (30), a patient-side drainage inlet (31) which has a conical shape and is designed with steps, which drainage inlet (31) extends approximately in axial alignment with the mouth of the patient-side end of the drainage tube (10) .

17. Patient-side attachment part (3) for use in a drainage tube unit according to one of Claims 1 to 16, in which the patient-side attachment part (3) has a drainage channel (37) with a patient-side drainage inlet (31) and with a pump-side drainage mouth for receiving a patient-side end of a drainage tube (10) and a pump-side service channel (35) for receiving an end of a service tube (11) that extends separate from the patient-side end of the drainage tube (10), the service channel (35) of the patient-side attachment part (3) having a smaller diameter than the drainage channel (37) of the patient-side attachment part (3) and with a connection channel (36) also being provided that connects the service channel (35) of the patient-side attachment part (3) to the drainage channel (37) of the patient-side attachment part (3), wherein a first end of the connection channel (36) leads into the drainage channel (37) of the patient-side attachment part (3) and a second end leads to the outside, which second end is able to be closed, and wherein the attachment part is produced in one piece by injection moulding.

18. Pump-side end-connector for use in a drainage tube unit according to one of Claims 1 to 16, in which the end-connector (2) has a main body (22) in which a drainage channel (24) of the end-connector (2) and a service channel (25) of the end-connector (2) extend, the drainage channel (24) having a pump-side drainage outlet (20), for connection to an associated suction device, and a patient-side drainage mouth for receiving a pump-side end of a drainage tube (10), the drainage outlet (20) being arranged on a different side of the main body (22) than the drainage mouth, and the service channel (25) of the end-connector (2) having a pump-side service inlet (23), for connection to an associated suction device, and a service mouth for receiving a pump-side end of the service tube (11) that extends separate from the patient-side end of the drainage tube (10), the service inlet (23) being arranged on a different side of the main body (22) than the service mouth, and the end-connector (2) being designed to be plugged into the suction device.

19. Pump-side end-connector according to Claim 18, in which the main body (22) has a substantially cuboid shape, the drainage outlet (20) is arranged substantially at right angles to the drainage mouth, and the service inlet (23) is arranged substantially at right angles to the service mouth.

20. Pump-side end-connector according to Claim 19, in which the drainage outlet (20) is arranged on an opposite side from the service inlet (23).

21. Drainage tube unit for aspirating body fluids by means of a suction pump, the tube unit comprising a drainage tube (10) for aspirating the body fluids and at least one service tube (11), each of them with a patient-side end and a pump-side end, the patient-side ends of the drainage tube (10) and of the service tube (11) being arranged in a common patient-side attachment part (3) according to Claim 17, wherein the patient-side end of the drainage tube (10) extends separate from the patient-side end of the service tube (11), and wherein the patient-side end of the drainage tube (10) opens into a drainage channel (37) of the patient-side attachment part (3) according to Claim 17 and the patient-side end of the service tube (11) opens into a service channel (35) of the patient-side attachment part (3) according to Claim 17.

## Revendications

1. Unité à tuyau de drainage destinée à l'aspiration de fluides corporels au moyen d'une pompe aspirante, la pompe aspirante présentant un tuyau de drainage (10) destiné à aspirer les fluides corporels et au moins un tuyau d'entretien (11) ayant chacun, respectivement, une extrémité côté patient et une extrémité côté pompe, les extrémités côté patient du tuyau de drainage (10) et du tuyau d'entretien (11) étant arrangées dans une pièce de raccord côté patient (3) commune, et les extrémités côté pompe du tuyau de drainage (10) et du tuyau d'entretien (11) étant arrangées dans un connecteur terminal côté pompe (2) commun, le connecteur terminal côté pompe (2) étant adapté pour être inséré dans un dispositif d'aspiration correspondant, les extrémités du tuyau de drainage (10) s'étendant séparément des extrémités du tuyau d'entretien (11), et l'extrémité côté patient du tuyau de drainage (10) débouchant dans un canal de drainage (37) de la pièce de raccord côté patient (3) et l'extrémité côté patient du tuyau de entretien (11) débouchant dans un canal d'entretien (35) de la pièce de raccord côté patient (3), et l'extrémité côté pompe du tuyau de drainage (10) débouchant dans un canal de drainage (24) du connecteur terminal côté pompe (2) et l'extrémité côté pompe du tuyau de entretien (11) débouchant dans un canal d'entretien (25) du connecteur terminal côté pompe (2).

2. Unité à tuyau de drainage selon la revendication 1, le connecteur terminal côté pompe (2) et/ou la pièce de raccord côté patient (3) étant réalisé intégralement.

3. Unité à tuyau de drainage selon une des revendications 1 ou 2, les extrémités côté patient et côté pompe du tuyau de drainage respectivement du tuyau d'entretien (11) s'étendant de manière parallèle l'une par rapport à l'autre débouchent dans le connecteur terminal (2) et la pièce de raccord côté patient (3).

4. Unité à tuyau de drainage selon une des revendications 1 à 3, les deux tuyaux étant deux tuyaux séparés à lumière unique.

5. Unité à tuyau de drainage selon une des revendications 1 à 4, les deux tuyaux débouchant dans au moins le connecteur terminal (2) et/ou la pièce de raccord côté patient (2) sur le même côté du connecteur terminal (2) ou de la pièce de raccord côté patient (2).

6. Unité à tuyau de drainage selon une des revendications 1 à 5, le canal de drainage (24) du connecteur terminal (2) présentent une bouche de drainage pour recevoir l'extrémité du tuyau de drainage côté pompe (10) et une sortie de drainage (20), la sortie de drainage (20) étant arrangée d'un autre côté que la bouche de drainage.

7. Unité à tuyau de drainage selon la revendication 6, le connecteur terminal (2) étant formé essentiellement en forme parallélépipède et la sortie de drainage (20) étant arrangée essentiellement de manière perpendiculaire à la bouche de drainage.

8. Unité à tuyau de drainage selon une des revendications 1 à 7, le canal de entretien (25) du connecteur terminal (2) présentant une bouche d'entretien pour recevoir l'extrémité côté pompe du tuyau d'entretien (11) et une entrée d'entretien (23), l'entrée d'entretien (23) étant arrangée d'un autre côté que la bouche d'entretien.

9. Unité à tuyau de drainage selon la revendication (8), le connecteur terminal (2) étant formé essentiellement en forme de parallélépipède et l'entrée d'entretien (23) étant arrangée essentiellement de manière perpendiculaire à la bouche d'entretien.

10. Unité à tuyau de drainage selon la revendication 8 et 9, l'entrée d'entretien (23) étant arrangée d'un côté opposé à la sortie de drainage (20) du connecteur terminal (2).

11. Unité à tuyau de drainage selon une des revendications 1 à 10, un filtre (6) étant arrangé dans le canal d'entretien (25) du connecteur terminal (2).

12. Unité à tuyau de drainage selon une des revendications 1 à 11, le connecteur terminal (2) étant formé de sorte à être tenu dans la pompe aspirante de manière amovible et solidaire.

13. Unité à tuyau de drainage selon une des revendications 1 à 12, le canal de drainage (37) et le canal d'entretien (35) de la pièce de raccord côté patient (3) étant connectés l'un à l'autre à travers un canal de connexion (37).

14. Unité à tuyau de drainage selon la revendication 13, le canal de connexion (37) menant dans le canal de drainage (36) à une première extrémité et vers l'extérieur à une deuxième extrémité, cette deuxième extrémité étant verouillable avec un couvercle de fermeture (32).

15. Unité à tuyau de drainage selon la revendication 14, le couvercle (32) étant monté intégralement à la pièce de raccord côté patient (3).

16. Unité à tuyau de drainage selon une des revendications 1 à 15, la pièce de raccord côté patient (3) présentant un corps de base (30) pour recevoir les extrémités des tuyaux de drainage et d'entretien (10, 11) et une entrée de drainage côté patient (31) étant formée sur le corps de base (30) de manière connectante et conique dégradée, l'entrée de drainage (31) s'étendant approximativement en alignement axial par rapport à la bouche de l'extrémité côté patient du tuyau de drainage (10).

17. Pièce de raccord côté patient (3) pour l'utilisation dans une unité à tuyau de drainage selon une des revendications 1 à 16, la pièce de raccord côté patient (3) présentant un canal de drainage (37) ayant une entrée de drainage côté patient (31) et une extrémité de tuyau d'entretien (11) ayant une bouche de drainage côté pompe pour recevoir une extrémité côté patient d'un tuyau de drainage (10) et ayant un canal d'entretien côté pompe (35) pour recevoir une extrémité de tuyau d'entretien (11) s'étendant séparément de l'extrémité côté patient du tuyau de drainage (10), le canal d'entretien (35) de la pièce de raccord côté patient (3) présentant un diamètre inférieur à celui du canal de drainage (37) de la pièce de raccord côté patient (3) et présentant d'avantage un canal de connexion (36), connectant le canal d'entretien (35) de la pièce de raccord côté patient (3) avec le canal de drainage (36) de la pièce de raccord côté patient (3), le canal de connexion (36) menant dans le canal de drainage (36) de la pièce de raccord côté patient (3) à une première extrémité et vers l'extérieur à une deuxième extrémité, la deuxième extrémité étant formée de manière verrouillable et la pièce de raccord étant fabriquée intégralement dans une procédé d'injection-moulage.

18. Connecteur terminal côté pompe pour l'utilisation dans une unité à tuyau de drainage selon une des revendications 1 à 16, le connecteur terminal (2) présentent un corps de base (22), dans lequel un canal de drainage (24) du connecteur terminal (2) et un canal d'entretien (25) du connecteur terminal (2) s'étendent, le canal de drainage (24) présentant une sortie de drainage côté pompe (20) pour la connexion avec un dispositif d'aspiration correspondant et une bouche de drainage côté patient pour recevoir d'une extrémité côté pompe d'un tuyau de drainage (10), la sortie de drainage (20) étant arrangée sur un autre côté du corps de base (22) que celui de la bouche de drainage, et le canal d'entretien (25) du connecteur terminal (2) présentant une entrée d'entretien côté pompe (23) pour la connexion avec un dispositif d'aspiration correspondant et une bouche d'entretien pour recevoir une extrémité côté pompe du tuyau d'entretien (11) s'étendant séparément à l'extrémité côté pompe du tuyau de drainage (10), l'entrée d'entretien (23) étant arrangée d'un autre côté du corps de base (22) que celui de la bouche d'entretien et le connecteur terminal (2) est formé pour être inséré dans le dispositif d'aspiration.

19. Connecteur terminal côté pompe selon la revendication 18, le corps de base (22) étant formé essentiellement en forme de parallélépipède, la sortie de drainage (20) étant essentiellement arrangée perpendiculairement à la bouche de drainage et l'entrée d'entretien (23) étant arrangée essentiellement perpendiculairement à la bouche d'entretien.

20. Connecteur terminal côté pompe selon la revendication 19, la sortie de drainage (20) étant arrangée d'un côté opposé à celui de l'entrée d'entretien (23).

21. Unité à tuyau de drainage destinée à l'aspiration de fluides corporels au moyen d'une pompe aspirante, l'unité à tuyau présentant un tuyau de drainage (10) destiné à aspirer les fluides corporels et au moins un tuyau d'entretien (11) présentant respectivement une extrémité côté patient et côté pompe, les extrémités côté patient du tuyau de drainage et du tuyau d'entretien (11) étant arrangés dans une pièce de raccord côté patient (3) commune selon la revendication 17, l'extrémité côté patient du tuyau de drainage (10) s'étendant séparément de l'extrémité côté patient du tuyau d'entretien (11), et l'extrémité côté patient du tuyau de drainage (10) débouchant dans un canal de drainage (37) de la pièce de raccord côté patient (3) selon la revendication 17 et l'extrémité côté patient du tuyau d'entretien (11) débouchant dans un canal d'entretien (35) de la pièce de raccord côté patient (3) selon la revendication 17.
